# EUROPÄISCHE PATENTANMELDUNG

(11) **EP 2 229 979 A1**
(43) Veröffentlichungstag der Anmeldung: **22.09.2010**
(21) Anmeldenummer: 09155532.6
(22) Anmeldetag: 18.03.2009
(51) Int. Cl.: A61N 5/06

(54) **Bestrahlung-Kühlkombination für die Anwendung in der photodynamischen Therapie**

(71) Anmelder: Hilty, Norbert, 9494 Schaan (LI)
(72) Erfinder: Hilty, Norbert, 9494 Schaan (LI)
(74) Vertreter: Kaminski Harmann

(57) **Zusammenfassung**

Die Erfindung betrifft eine Bestrahlungs-Kühl-Kombination für die Anwendung in der photodynamischen Therapie mit einem Bestrahlungselement (120), einem für die emittierte Strahlung in einer Strahlungsdurchtrittsrichtung durchlässigen Kühlelement (130), wobei dieses zur Anbringung zwischen dem Bestrahlungselement (120) und einer Behandlungsfläche (160) eines Patienten ausgebildet ist. Das Kühlelement (130) weist dabei eine kühlbare optische Durchtrittsfläche als eine Anlage an die Behandlungsfläche (160) auf.

Weitere Komponenten der Erfindung sind eine erste Fixierungsvorrichtung (150) zur definierten Positionierung der Bestrahlungs-Kühl-Kombination gegenüber der Behandlungsfläche (160), eine zweite Fixierungsvorrichtung (140) zur Anbringung des Bestrahlungselements (120) am Kühlelement (130), eine Steuereinheit (110) sowie ein Netzteil (100).

Die Erfindung stellt ein wirkungsvolles Schmerzmanagement im Rahmen der photodynamischen Therapie in Verbindung mit einem mobilen Behandlungssystem sowie größeren Behandlungsflächen mit einem homogenisierten Strahlungsfeld bereit.

## Beschreibung

Die Erfindung betrifft eine Bestrahlungs-Kühl-Kombination für eine Anwendung in der Photodynamischen Therapie nach dem Oberbegriff des Anspruchs 1 und ein Bestrahlungsverfahren zum Bereitstellen eines homogenisierten Strahlungsfeldes an einer gekühlten optischen Durchtrittsfläche nach dem Oberbegriff des Anspruchs 14.

Unter der Photodynamischen Therapie (PDT) versteht man ein Verfahren zur Behandlung von Tumoren und anderen Gewebeveränderungen mit Licht in Kombination mit einer lichtempfindlichen Substanz - einem so genannten Photosensibilisator oder Photosensitizer - und im Gewebe vorhandenem Sauerstoff. Dazu wird dem Patienten ein solcher Sensibilisator oder einer seiner Stoffwechselvorläufer verabreicht, der sich selektiv im Tumor anreichert. Nach einer gewissen Wartezeit wird anschließend der Tumor und das ihn umgebende gesunde Gewebe mit Licht geeigneter Wellenlänge bestrahlt. Dabei werden durch photophysikalische Prozesse zytotoxische Substanzen erzeugt, die aufgrund der Tumorselektivität des Sensibilisators gezielt den Tumor schädigen.

Als Photosensibilisatoren finden überwiegend Porphyrine Verwendung, die sich bei Bestrahlung mit rotem Licht mit einer Wellenlänge von 630 nm bis 635 nm aktivieren lassen. Gegenwärtig wird vor allem 5-Aminolävulinsäure (ALA) oder deren Methylester (MALA) eingesetzt, ein Stoffwechselvorläufer des Protoporphyrins IX, der selektiv in den Tumorzellen eine Porphyrinsynthese anregt. Neuere Sensibilisatoren lassen sich bei noch größeren Wellenlängen anregen mit dem Vorteil einer etwas größeren Tiefenwirkung der PDT, da Licht mit größeren Wellenlängen eine größere Eindringtiefe in die Haut aufweist.

Die schädigende Wirkung auf das Tumorgewebe beruht in der Regel auf der Entstehung von energetisch angeregtem und sehr reaktivem Singulett-Sauerstoff, der das umliegende kranke Gewebe oxidativ zerstört.

Als geeignete Lichtquellen zum Einsatz im Rahmen der PDT haben sich sowohl inkohärente Lampen, beispielsweise Xenon- oder Halogenlampen, als auch Lasersysteme und Leuchtdioden (LED) erwiesen. Eine Reihe verschiedener Lichtquellen für die PDT ist erhältlich und gelangt zum Einsatz.

Im Folgenden wird für den Wellenlängenbereich des sichtbaren Lichts sowie der nahen Ultraviolett- und Infrarotstrahlung allgemein der Ausdruck "Licht" verwendet.

Ein besonders großes Problem bei der Anwendung der PDT ist der beim Patienten unmittelbar nach Bestrahlungsbeginn einsetzende und teilweise als heftig empfundene Schmerz. Einige Anwender versuchen, bereits vor der Bestrahlung mittels einer lokalen oder systemischen Schmerzbehandlung Abhilfe zu schaffen, jedoch hat sich die zwischenzeitliche Kühlung der Behandlungsfläche (Hautoberfläche des Patienten) mit Eispackungen, Kältespray (z.B. Ethylchlorid), Gelpackungen, Cool Packs oder durch Luftkühlung mittels einer Ventilationsvorrichtung bisher als effizienteste Methode zur Schmerzreduktion herausgestellt.

Durch die Bestrahlung mit Licht kommt es lokal zu einer Erwärmung der Behandlungsfläche und des sich darunter befindlichen Gewebes. Die Schmerzleitung in den Nerven wird durch Stimulation eines Rezeptors (TRPV-1) initiiert, der unter normalen Bedingungen bei 41°C, nach einer Applikation des Photosensibilisators aber bereits bei 33°C aktiviert wird.

Die während der Bestrahlung auftretenden Schmerzen erfordern eine Intervention des die Behandlung durchführenden medizinischen Personals in Form einer zeitweiligen optischen Abdeckung des Behandlungsareals bzw. nötigenfalls einer Unterbrechung der Anwendung und Auflegen von Kühlmitteln. Hierdurch kommt es zu verlängerten Behandlungszeiten oder gar zum Abbruch der Bestrahlung.

Die aus dem Stand der Technik bekannten Apparate und Geräte verfügen nicht über ein integriertes, mit der Hautoberfläche in direktem Kontakt stehendes System zum Schmerzmanagement. Ventilatoren im Gehäuse der PDT-Lampe, wie sie beispielsweise im Modell "Omnilux" der Firma "Photo Therapeutics Ltd." (Altrincham, UK) realisiert wurden, sollen der Schmerzreduktion dienen, erweisen sich aber in der praktischen Anwendung als zu wenig effizient, um eine ausreichende Schmerzlinderung beim Patienten zu erzielen.

Gegenwärtig für die PDT eingesetzte Geräte besitzen zumeist ein Bestrahlungselement, das an einem Gelenkarm angebracht ist, z.B. die Modelle "Aktilite CL16" und "Aktilite CL128" von der Firma "Photocure ASA" (Oslo, Norwegen) oder "Waldmann 1200L" und "Waldmann 450L" von der Firma "Herbert Waldmann GmbH & Co. KG" (Villingen-Schwenningen, Deutschland). Der Gelenkarm seinerseits befindet sich auf einem fahrbaren Stativ oder kann mittels einer Halterung beispielsweise an einer Tischplatte befestigt werden.

In Verbindung mit dem massiven Netzteil und Steuergerät sowie dem Gelenkarm bildet das Bestrahlungselement einen unhandlichen und ortsgebundenen Apparat, der im wesentlichen nur für die Anwendung in medizinischen Behandlungsräumen geeignet ist. Der Patient wird in einer ruhenden, beispielsweise liegenden oder sitzenden, Haltung der Behandlung unterzogen. Die Mobilität des Patienten ist damit während der Behandlung vollständig eingeschränkt.

Einen weiteren Nachteil bei der Anwendung des obigen Gerätetyps für die PDT stellt die Inhomogenität des auf die Behandlungsfläche auftreffenden Strahlungsfelds dar. Die im wesentlichen planare Anordnung der diskreten Lichtquellen des Bestrahlungselements bewirkt auf der meist konvex gewölbten Behandlungsfläche eine ungleichmäßige Einstrahlung, d.h. unterschiedliche Flächensegmente sind einer unterschiedlichen Lichtdosis ausgesetzt. Dieser Effekt wirkt sich umso mehr aus, je größer das zu behandelnde Areal ist.

Für das Gerät "Aktilite CL16" wird aus oben angeführten Gründen lediglich eine Behandlungsfläche von ca. 20 cm² angegeben.

Das Bestrahlungselement des obigen Gerätetyps liegt zudem nicht an der Behandlungsfläche an, z.B. der Hautoberfläche des Patienten, sondern befindet sich in einem von dosimetrischen Parametern abhängigen Abstand von dieser. Aufgrund dieses Abstands fällt ein Teil des ausgesandten Lichtkegels nicht mehr auf das Behandlungsareal, sondern auf nicht zu therapierende Flächen, die dann abgedeckt werden müssen, oder entweicht gänzlich. Die Folgen der teilweise ungenutzten Lichtleistung sind ein erhöhter Energiebedarf und damit erhöhte Energiekosten.

Ein zusätzlicher Nachteil dieses Gerätetyps ergibt sich aus dem Umstand, dass diese Geräte nur ein Bestrahlungselement, meist in Form einer Leiste von diskreten Lichtquellen, besitzen. Damit können nicht mehrere, von einander entfernte, Areale synchron behandelt werden. Hierzu muss das Gerät jeweils neu justiert und die Behandlung wieder neu begonnen werden.

Das Dokument US 6,290,713 offenbart eine flexible Lichtmatte zur Anwendung in der PDT - vor allem zur Behandlung von Neugeborenen gegen postnatale Hepatitis - mit einer Vielzahl von integrierten Leuchtdioden, einer Diffusorschicht zur Streuung des emittierten Lichts und einer Kühlvorrichtung mit Kühlkanälen zur Ableitung der entstehenden Wärme. Die Lichtmatte kann aufgrund ihrer Flexibilität um ein zu behandelndes Körperteil eines Erwachsenen oder den Rumpf eines Kleinkindes gewickelt werden.

Die Kühlvorrichtung kann sowohl mit einem gasförmigen (z.B. Luft) als auch mit einem flüssigen durchströmenden Medium betrieben werden, dient aber im wesentlichen nur zum Abtransport der durch die Lampen selbst entstehenden Hitze. Eine direkte Kühlung der durch die eingedrungene Strahlung aufgeheizten oberen Haut- und Gewebeschichten findet dagegen nicht statt.

Daneben bewirkt die Verbindung der Kühlung der Lichtmatte mit einer externen Pumpe für das Kühlmedium über ein Schlauchsystem die Abhängigkeit von einem externen Gerät während der Behandlung und führt damit zur weitgehenden Immobilität des Patienten.

Die Diffusorschicht zwischen den Lichtquellen und der Behandlungsfläche enthält kleine Lack- oder Pulverpartikel, an denen das durch die Schicht durchtretende Licht stark gestreut wird. Die neben der Streuwirkung auftretende Absorption des Lichts durch die Partikel führt zu einem nicht zu vernachlässigenden Leistungsverlust der Strahlung auf der Strecke zwischen Lichtquelle und Behandlungsfläche.

Aus US 7,304,201 ist eine selbstklebende tragbare Bandage für die PDT zur Anbringung an ein bestimmtes Areal bekannt, die eine flexible Lichtemitterschicht und einen austauschbaren lichtdurchlässigen Wundverband aufweist und sich der Form der Behandlungsfläche anpassen kann. Mittels einer auf ihrer Innenseite reflektierenden Außenhülle wird das Licht zurückgeworfen, eine weitere Diffusionswirkung ist jedoch nicht vorhanden. Die Bandage besitzt zudem keine Kühlungsvorrichtung.

US 2008/0039906 offenbart einen lichttherapeutischen Behandlungsapparat zur körperexternen und -internen Anwendung mit einer Bestrahlungseinheit und einem integrierten Kühlschlauchsystem zur Abführung der durch die Lichtquellen entstehenden Hitze durch ein flüssiges Kühlmedium. Eine aktive Kühlung der bestrahlten Haut- oder Gewebeschichten findet ebenso wenig statt wie eine strahlungsleitende Einwirkung auf das emittierte Licht.

Die Form der Bestrahlungseinheit ist so ausgebildet, dass sie der Behandlungsfläche vorangepasst ist. Für das Kühlschlauchsystem ist wiederum eine externe Versorgungseinheit - beispielsweise eine Pumpe - Voraussetzung.

Aus dem Dokument US 6,454,789 ist ein vom Patienten durch einen Gurt tragbarer PDT-Apparat für eine Anwendung innerhalb des Körpers mittels einer Kanüle und einer darin verlaufenden Lichtfaser bekannt. Durch die Trageweise und die Energieversorgung mittels einer Batterie gewinnt der Patient eine weitgehende Mobilität. Ein Kühlungssystem und eine Vorrichtung zu Homogenisierung des Strahlungsfelds sind nicht vorhanden.

Eine Aufgabe der vorliegenden Erfindung ist die Bereitstellung eines zur Anwendung in der PDT ausgebildeten Systems mit integriertem Schmerzmanagement zur Reduktion und Vermeidung von Schmerzen für den Patienten während einer Bestrahlungsbehandlung.

Das Schmerzmanagement des Systems soll eine kontinuierliche und schmerzfreie Behandlung ohne Unterbrechungen gewährleisten.

Eine weitere Aufgabe der Erfindung ist die Bereitstellung eines im wesentlichen isotropen Strahlungsfeldes für die PDT, das auf jedem Flächensegment der Behandlungsfläche mit nahezu derselben Intensität und demselben Strahlquerschnitt einwirken kann.

Weiterhin ist es vorteilhaft, wenn durch die dazu eingesetzten Mittel nur ein geringer Leistungsverlust der beeinflussten Strahlung von maximal 5% resultiert.

Für die Einstrahlung auf eine Behandlungsfläche soll ein homogenisiertes Strahlungsfeld bereitgestellt werden, dessen Austrittsfläche mit den derzeit üblichen Behandlungsflächen von ca. 20 cm² vergleichbar oder größer ist.

Das Wellenlängenspektrum der angewandten Strahlung ist gemäß Aufgabe der Erfindung flexibel im zur medizinischen Anwendung im Rahmen der PDT geeigneten Bereich, wobei das Wellenlängenspektrum für eine diskrete Behandlung dagegen klar definiert ist.

Eine weitere Aufgabe der Erfindung besteht in der Möglichkeit zur synchronen Behandlung von mehreren, voneinander entfernten Arealen.

Zusätzlich soll die vorliegende Erfindung eine Bestrahlungsanwendung im Rahmen der PDT weitgehend unabhängig von medizinischen Behandlungsräumlichkeiten machen.

Diese Aufgaben werden erfindungsgemäß durch die Verwirklichung der Gegenstände der unabhängigen Ansprüche 1 und 14 gelöst. Merkmale, die die Erfindung in alternativer oder vorteilhafter Weise weiterbilden, sind den abhängigen Patentansprüchen zu entnehmen.

Erfindungsgemäß ist die Bestrahlungs- und Kühleinheit bzw. eine Bestrahlungs-Kühl-Kombination als modulares System mit vier Hauptkomponenten ausgebildet. Dabei wird erfindungsgemäß durch das Zusammenwirken der Komponenten eine Homogenisierung des Strahlungsfeldes bewirkt.

Eine solche Homogenisierung besteht darin, dass an der gekühlten Durchtrittsfläche ein Strahlungsfeld bereitgestellt wird, das eine minimale innere Struktur aufweist, d.h. keine oder nur minimale ortsabhängige Variationen der Intensität. Da die von den einzelnen Strahlungsquellen emittierte Strahlung somit diskrete Quellpunkte aufweist und eine zumeist große Divergenz besitzt, muss die durch die Erzeugung aufgeprägte innere Struktur verringert oder beseitigt werden.

Durch die Divergenz entstehen je nach Position unterschiedliche Auftreffwinkel auf die Behandlungsoberfläche, die zu unterschiedlichen Leuchtdichten führen, so dass ein möglichst gleichförmiger Einfallswinkel angestrebt wird. Diese Divergenzänderung kann durch einen Linseneffekt erreicht werden, der erfindungsgemäß durch das kühlende Medium bzw. das zur Aufnahme verwendete Behältnis bewirkt wird. Obgleich aufgrund der Dimensionierung und dem Abstand von Quelle zu Behandlungsfläche in den seltensten Fällen eine vollständige Kollimierung erreicht werden kann, so zeigt bereits eine geringe Linsenwirkung in der Praxis schon eine Verbesserung der Bestrahlungsqualität.

Ein zweiter zur Homogenisierung genutzter Effekt besteht im Zurückführen ansonsten aus dem Strahlgang austretender Strahlungsanteile durch Reflektion an der Wandung. Beide Effekte führen im Zusammenwirken zu einem homogeneren Strahlungsfeld, bei dem durch Reflektion und Divergenzänderung bzw. Fokussierung zudem auch die Verluste verringert sind, so dass neben dem verbesserten Wirkungsgrad der Anordnung in der Folge auch die quellenseitig entstehende thermische Belastung verringert werden kann.

Die Hauptkomponenten der erfindungsgemäßen Bestrahlungs-Kühl-Kombination sind im einzelnen: mindestens ein Bestrahlungselement zur Emission des im Rahmen einer PDT angewandten Lichts, mindestens ein Kühlelement zur Schmerzreduktion beim PDT-Patienten, das zugleich durch geeignete Ausbildung das Auftreffen eines homogenisierten Strahlungsfelds auf der Behandlungsfläche des Patienten gewährleistet, sowie eine Steuereinheit und ein Netzteil.

Bestrahlungselement, Steuereinheit und/oder Netzteil bilden einen funktionalen Verband und können dabei sowohl als räumlich getrennte Geräte als auch als eine modulare Einheit ausgebildet sein.

Als weitere Komponenten weist das System eine erste Fixierungsvorrichtung zur definierten Positionierung der Bestrahlungs-Kühl-Kombination gegenüber der Behandlungsfläche und eine zweite Fixierungsvorrichtung zur Anbringung des Bestrahlungselements an das Kühlelement auf. Beide Fixierungsvorrichtungen sind grundsätzlich auch gemeinsam in einer einzigen Fixierungsvorrichtung realisierbar.

Im Arbeitseinsatz im Rahmen einer PDT-Anwendung liegt das Kühlelement auf der einen Seite mit einer kühlbaren optischen Durchtrittsfläche an der Behandlungsfläche an, während eine zweite optische Durchtrittsfläche an der Austrittsseite der emittierten Strahlung des Bestrahlungselements anliegt.

Das Bestrahlungselement ist als eine im wesentlichen planare Anordnung von diskreten Lichtquellen ausgebildet, die geeignet sind für die kontinuierliche und/oder gepulste Emission einer Strahlung mindestens einer Wellenlänge zur Anwendung in der PDT. In der PDT können prinzipiell sichtbares Licht, nahes Infrarot und nahes Ultraviolett zum Einsatz gelangen.

Eine bevorzugte Ausführung der Erfindung beinhaltet die Ausbildung des Bestrahlungselements als LED-Leiste mit Vorsatzlinsen. Das Abführen der durch die Lichtquellen unmittelbar entstandenen Wärme erfolgt mittels einer Lüftungsvorrichtung.

Das Bestrahlungselement ist zur Anbringung mit seiner Strahlungsaustrittsseite an das Kühlelement - insbesondere mittels der zweiten Fixierungsvorrichtung - geeignet. Eine bevorzugte Ausführung der Erfindung beinhaltet die Ausbildung der zweiten Fixierungsvorrichtung als Spange oder Rahmen.

Die dosimetrische Steuerung, beispielsweise die Einstellung der Zeitdauer und Intensität einer Bestrahlung, sowie die Auswahl einer oder mehrerer Wellenlängen der eingesetzten Strahlung des Bestrahlungselements erfolgt mittels einer Steuereinheit. Eine Steuereinheit kann dabei in einer Ausführung der Erfindung mehrere Bestrahlungselemente abhängig oder unabhängig voneinander steuern.

Das Netzteil zur Energieversorgung ist für eine mobile PDT-Anwendung geeignet und ist in einer bevorzugten Ausführung der Erfindung als Batterie ausgebildet.

Das Kühlelement weist eine Hülle und ein deformierbares - insbesondere flüssiges oder gelartiges - Füllmedium auf und ist in einer Durchtrittsrichtung der emittierten Strahlung für diese durchlässig.

Als Füllmedium kommen solche Stoffe in Frage, die aufgrund ihrer Wärmekapazität zum Vorgekühltwerden geeignet sind. Neben der Durchlässigkeit für Licht muss das Füllmedium einen entsprechend niedrigen Fließpunkt aufweisen, so dass es ohne Verfestigung im Kühlschrank bei nahe 0°C, insbesondere bei 4-6°, aufbewahrbar ist.

Eine bevorzugte Ausführung der Erfindung beinhaltet die Verwendung von viskosen medizinischen Weißölen als Füllmedium für das Kühlelement, beispielsweise "Ondina 941" von der Firma "Shell".

Die Hülle des Kühlelements weist eine oder mehrere Seitenflächen, die im wesentlichen in Durchtrittsrichtung des Lichts orientiert sind und das Licht auf ihrer Innenseite im wesentlichen reflektieren, sowie zwei Durchtrittsflächen auf, wobei eine Durchtrittsfläche die zum Anliegen an die Behandlungsfläche geeignete kühlbare optische Durchtrittsfläche darstellt.

Die Durchtrittsflächen des Kühlelements können sowohl rund als auch eckig ausgebildet sein und weisen bauartbedingt eine höhere Flexibilität als die Seitenwände auf. Letztere bewahren, beispielsweise aufgrund ihrer im Vergleich zu den Durchtrittsflächen größeren Wandstärke oder der Verwendung eines an sich festeren Materials, unter dem Anliegen eines zusätzlichen äußeren Drucks - insbesondere nach dem Anbringen an die Behandlungsfläche - im wesentlichen ihre Formstabilität. Auf diese Weise ist ein im wesentlichen stabiler Abstand zwischen den Durchtrittsflächen mittels der Seitenwandhöhe definierbar, während die flexible kühlbare Durchtrittsfläche an die Form der Behandlungsfläche anpassbar ist.

Kommt es verursacht durch das Anliegen des Kühlelements an der Behandlungsfläche zu dessen Deformation, wirkt das Kühlelement mit seinem Füllmedium divergenzverändernd auf das emittierte Licht ein (Linseneffekt). Gemeinsam mit der Reflektion des Lichts an der Innenseite der Seitenwände wird eine Durchmischung und Isotropie der durchtretenden Strahlung bewirkt. Das an der kühlbaren optischen Durchtrittsfläche austretende und auf die Behandlungsfläche einwirkende Licht weist daher eine weitgehende Homogenisierung auf.

Aufgrund der Flexibilität der Durchtrittsfläche und des aus ihr austretenden und infolge obiger Effekte homogenisierten Strahlungsfeldes sind nunmehr größere Behandlungsflächen pro Bestrahlungseinheit zugänglich.

Das Kühlelement wird vor einer PDT-Behandlung des Patienten in einer Kühlvorrichtung, beispielsweise einem Kühlschrank, auf eine Temperatur von etwa 4-6°C vorgekühlt und mit dieser Temperatur, die für den Patienten noch als angenehm empfunden wird, und mit seiner kühlbaren optischen Durchtrittsfläche an die Behandlungsfläche - insbesondere die Haut - angelegt. Während des kompletten Bestrahlungsvorgangs, der im Schnitt bis zu 20 Minuten beträgt, verbleibt das Kühlelement an die Behandlungsfläche angelegt und bewirkt auf diese Weise, dass sich mittels der kontinuierlichen Kühlung die behandelte Fläche und das darunter liegende Gewebe nicht bis zu einer vom Patienten als unangenehm oder gar schmerzhaft wahrgenommenen Temperatur erwärmen können.

Die zur definierten Positionierung der Bestrahlungs-Kühl-Kombination gegenüber der Behandlungsfläche geeignete erste Fixierungsvorrichtung ist in einer bevorzugten Ausführung der Erfindung als verschließbarer Gurt - insbesondere mit einem Klettverschluss - ausgebildet. Am Gurt sind unmittelbar eines oder mehrere Kühlelemente angebracht, wobei der Gurt für den Patienten zum Tragen am Körper geeignet ist. Weiterhin sind mehrere Gurte mit unmittelbar daran angebrachten Kühlelementen synchron am Körper des Patienten anlegbar und von diesem tragbar, so dass eine synchrone PDT-Behandlung voneinander entfernter Behandlungsflächen durchführbar ist.

Die Ausbildung der erfindungsgemäßen Bestrahlungs-Kühl-Kombination als tragbares und somit mobiles System gewährleistet eine weitgehende Mobilität des Patienten. Durch die Kombination der Mobilität mit dem integrierten Schmerzmanagementsystem benötigt der Patient im wesentlichen keine Betreuung durch medizinisches Personal und ist unabhängig von Behandlungsräumlichkeiten. Die PDT-Anwendung ist durch den Patienten selbst an einem beliebigen Ort und ohne Aufsicht durchführbar.

Die bevorzugte Ausführung der Erfindung beinhaltet die Verwendung von Folien aus thermoplastischem Polyurethanester oder -ether für die Hülle des Kühlelements und den Fixierungsgurt, beispielsweise "Sarna Plastec 2201" oder "Sarna Plastec 4201" der Firma "Sarna Plastec" (Samen, Schweiz).

Das Kühlelement ("Gel-Pad") ist hierbei auf einem Polymer-Gurt aufgeschweißt, der einen Klettverschluss (Velcro male/female), geeignet zur Fixierung des Gel-Pads zusammen mit dem Bestrahlungselement auf der Körperoberfläche, aufweist.

Eine mechanische Fixierung der erfindungsgemäßen Vorrichtung an der Behandlungsfläche ist sowohl unmittelbar am Kühlelement als auch mittelbar am Bestrahlungselement realisierbar, letzteres bewirkt dabei mittels der zweiten Fixierungsvorrichtung zugleich die definierte Positionierung des Kühlelements zur Behandlungsfläche.

Als weitere Ausführungen der Erfindung sind beispielsweise die Fixierung des Kühlelements mittels eines Adhäsivverbands unmittelbar auf der Behandlungsfläche sowie die Fixierung von Kühl- und Bestrahlungselement mittels eines Wickelverbands denkbar.

Durch die Verwendung der erfindungsgemäßen Bestrahlungs-Kühl-Kombination in der PDT ist ein wirkungsvolles Schmerzmanagement für den Patienten unter weitgehender Befreiung von durch die Behandlung verursachten Schmerzen verfügbar. In Verbindung damit wird ein kompaktes mobiles PDT-Behandlungssystem mit vollständiger Bewegungsfreiheit und Unabhängigkeit für den Patienten sowie größeren Behandlungsflächen pro Bestrahlungseinheit bereitgestellt.

Die erfindungsgemäße Vorrichtung und das erfindungsgemäße Verfahren werden nachfolgend anhand der Zeichnungen näher beschrieben. Im Einzelnen zeigen:
- Fig. 1: eine an der Behandlungsfläche anliegende erfindungsgemäße Bestrahlungs-Kühl-Kombination (schematisch) in der Schnittansicht;
- Fig. 2: eine erfindungsgemäße Bestrahlungs-Kühl- Kombination in ihrem modularen Aufbau;
- Fig. 3a/b: eine schematische Darstellung eines Bestrahlungs- elements mit einer LED-Lichtleiste;
- Fig. 4a/b: eine schematische Darstellung eines geöffneten Bestrahlungselements ohne Abdeckung
- Fig. 5: ein Kühlelement in der Schrägansicht;
- Fig. 6: ein Kühlelement in Blickrichtung der Durchtritts- richtung der Strahlung;
- Fig. 7a/b: ein Kühlelement in der Seitenansicht;
- Fig. 8: ein Kühlelement mit seinen deformierbaren Durch- trittsflächen für die Strahlung
- Fig. 9a: einen möglichen Verlauf der Strahlung innerhalb des Kühlelements;
- Fig. 9b: einen Verlauf der Strahlung ohne zwischengeschal- tete optische Mittel;
- Fig. 9c: eine Beeinflussung des Strahlengangs durch die kollimierende Wirkung des Kühlelements;
- Fig. 9d: eine Beeinflussung des Strahlengangs durch die Reflektion der Strahlung an den Seitenwänden des Kühlelements;
- Fig. 10: ein auf einem Fixierungsgurt angebrachtes Kühl- element in der Schrägansicht;
- Fig. 11: ein auf einem Fixierungsgurt angebrachtes Kühl- element in der Draufsicht (in Durchtrittsrichtung der Strahlung);
- Fig. 12: ein auf einem Fixierungsgurt angebrachtes Kühl- element in der Seitenansicht.

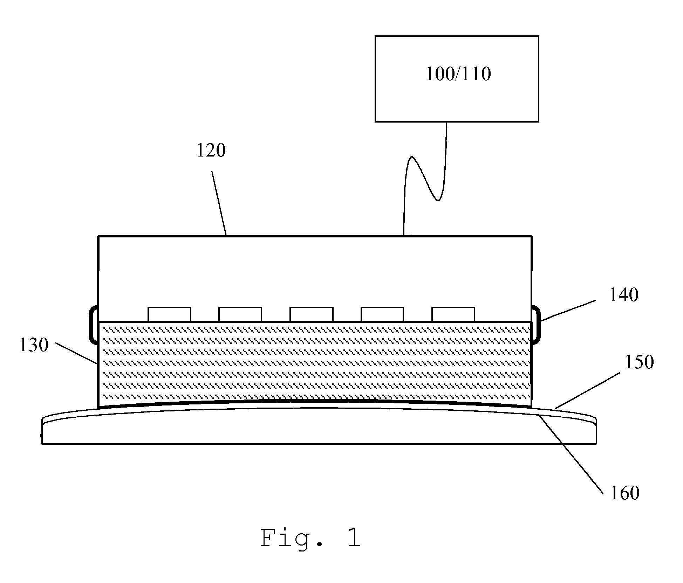

**Figur 1** stellt eine an der Behandlungsfläche angelegte erfindungsgemäße Bestrahlungs-Kühl-Kombination dar. Deren Anbringung an der Behandlungsfläche erfolgt mittels einer ersten Fixierungsvorrichtung.

Das mit Netzteil 100 und Steuereinheit 110 verbundene Bestrahlungselement 120 ist an dem Kühlelement 130 mittels einer zweiten Fixierungsvorrichtung 140 angebracht. Das Kühlelement 130 seinerseits ist an einem Tragegurt 150 angeschweißt, der in dieser Ausführung der Erfindung die obige erste Fixierungsvorrichtung darstellt, und dieser an der Behandlungsfläche 160 fixiert.

Die zweite Fixierungsvorrichtung 140 kann beispielsweise als Spange oder Rahmen ausgebildet sein.

Das Kühlelement 130 ist bauartbedingt deformierbar und an die Behandlungsfläche 160 anpassbar.

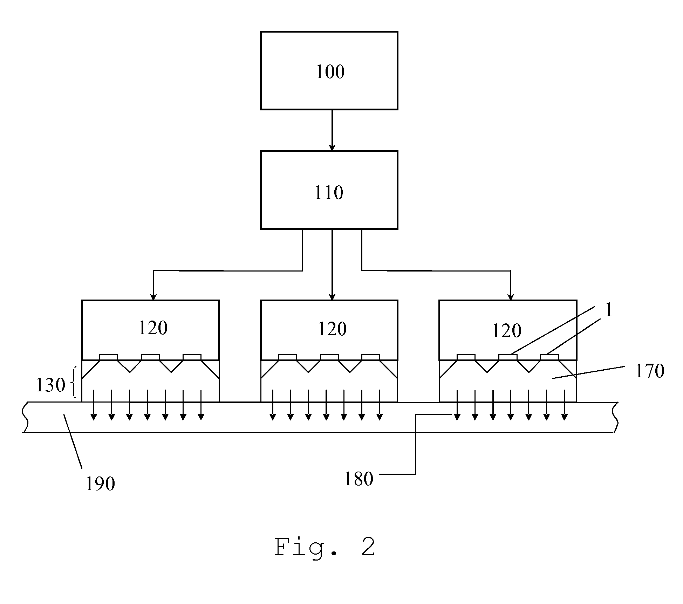

Aus **Figur 2** ist in schematischer Darstellung der modulare Aufbau der erfindungsgemäßen Bestrahlungs-Kühl-Kombination zu entnehmen.

Über das Netzteil 100 wird die Steuereinheit 110 mit Energie versorgt, die eine oder mehrere Bestrahlungselemente 120 dosimetrisch steuert. Die Bestrahlungselemente 120 ihrerseits sind mittels einer Fixierungsvorrichtung (nicht dargestellt) an den Kühlelementen 130 angebracht.

Die Leuchtdioden 1 der Bestrahlungseinheit 130 emittieren jeweils ein Strahlungsfeld 170. Dessen Strahlung wird innerhalb des Kühlelements 130 aufgrund von Reflektion und Linseneffekt durchmischt, so dass eine im wesentlichen homogenisierte Strahlung 180 auf die Behandlungsfläche auftrifft und in das darunter liegende Gewebe 190 eindringt.

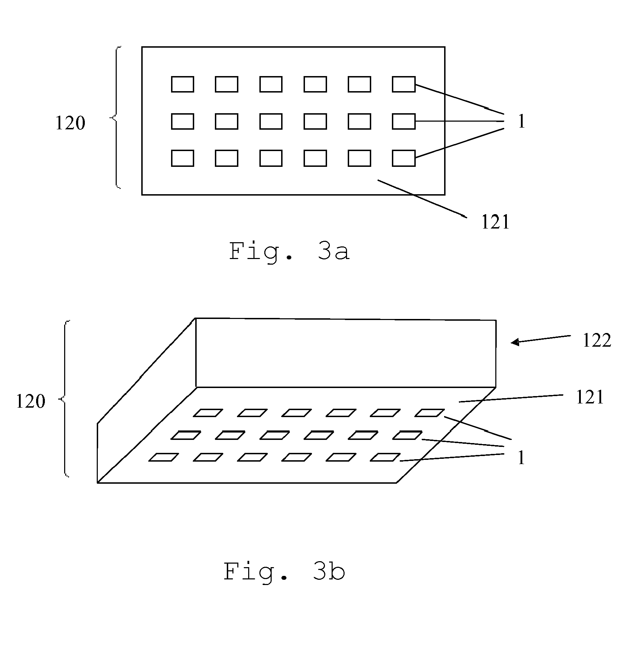

**Figur 3a** und **3b** zeigen schematisch ein Bestrahlungselement mit einer Leiste von Leuchtdioden (LED) 1. **Figur 3a** zeigt die Strahlungsaustrittsfläche 121 mit der LED-Leiste in der Draufsicht, **Figur 3b** das Bestrahlungselement 120 mit seinem Gehäuse 122 sowie der Strahlungsaustrittsfläche 121 mit den LEDs 1 in der Schrägansicht. Die Lichtquellen in der LED-Leiste sind dabei matrixförmig angeordnet.

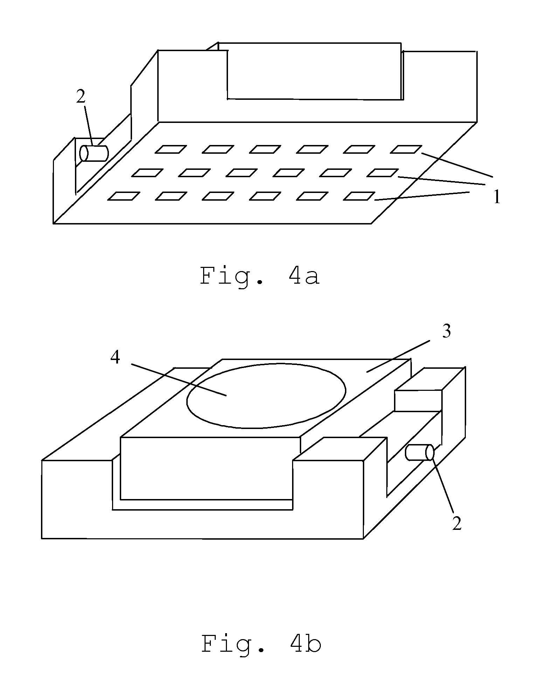

**Figur 4a** und **4b** zeigen ein geöffnetes Bestrahlungselement nach dem Entfernen der Abdeckung. Neben den LEDs 1 weist dieses einen Stromanschluss 2 sowie einen Kühlkörper 3 mit Lüftereinlass 4 auf. In **Figur 4a** sieht der Betrachter schräg in Richtung der Strahlungsaustrittsfläche, in **Figur 4b** schräg in Richtung der abgenommenen Abdeckung auf das Bestrahlungselement.

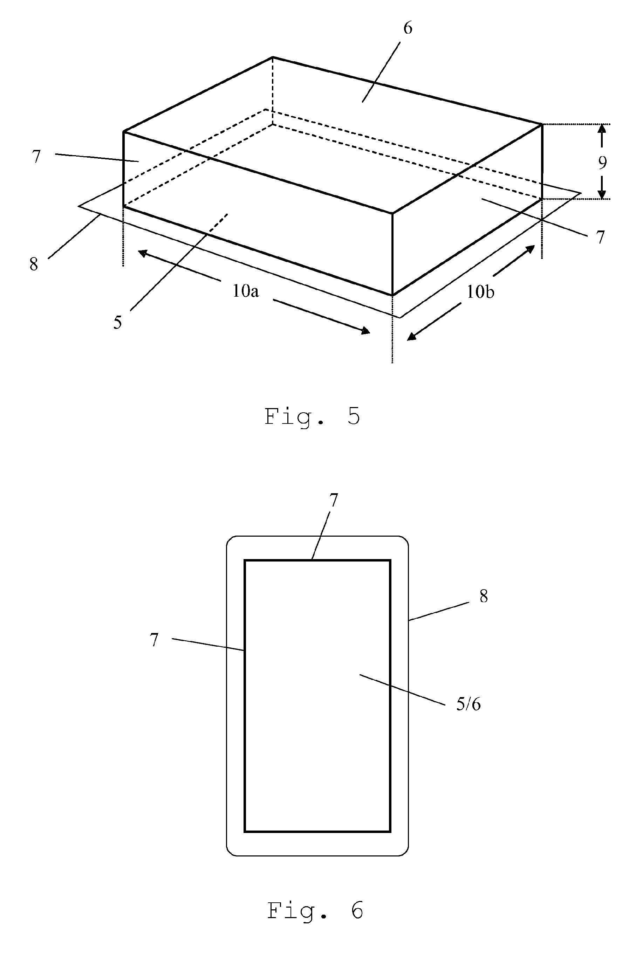

Das in **Figur 5** dargestellte Kühlelement ist ausgebildet als Gel-Pad mit einer Kunststoffhülle, die aus den optischen Durchtrittsflächen 5 und 6 sowie den Seitenflächen 7 besteht. Die beiden Durchtrittsflächen 5 und 6 besitzen dabei im wesentlichen dieselbe Grundfläche und sind im wesentlichen parallel angeordnet.

Bei dem abgebildeten Kühlelement handelt es sich um ein solches mit einer rechteckigen Form der Durchtrittsflächen. Diese können prinzipiell jedoch auch als andere Vielecke sowie als kreisförmige oder elliptische Formen ausgebildet sein.

Die Durchtrittsfläche 5 entspricht der kühlbaren optischen Durchtrittsfläche und ist zum Anschweißen an einen Kunststoff-Tragegurt ausgebildet. Dazu ist - wie in **Fig. 5** abgebildet - die kühlbare optische Durchtrittsfläche 5 optional mit einer Umrandung 8 versehbar.

Die zweite Durchtrittsfläche 6 liegt während einer PDT-Behandlung an der Strahlungsaustrittsfläche des Bestrahlungselements an.

Die jeweiligen Seitenflächen 7 weisen dieselbe Höhe 9 auf, können prinzipiell aber sowohl die gleiche als auch unterschiedliche Breiten 10a und 10b und somit verschiedene Flächeninhalte besitzen. Die Höhe 9 der Seitenflächen 7 ist dabei kleiner als die kürzeste geometrische Achse der Durchtrittsflächen. Diese Achse stellt bei einer rechteckigen Durchtrittsfläche die kürzere Breite 10b der Seitenflächen 7, im Falle von beispielsweise ellipsenförmigen Durchtrittsflächen die Nebenachse der Ellipse dar.

Zur Erhöhung des Reflektionsvermögens können die Seitenflächen des Kühlelements poliert, geschliffen, beschichtet oder bedampft sein.

**Figur 6** zeigt das Kühlelement mit den beiden Durchtrittsflächen 5 und 6, der Umrandung 8 der Fläche 5 und den Seitenflächen 7 in der Durchtrittsrichtung der emittierten Strahlung.

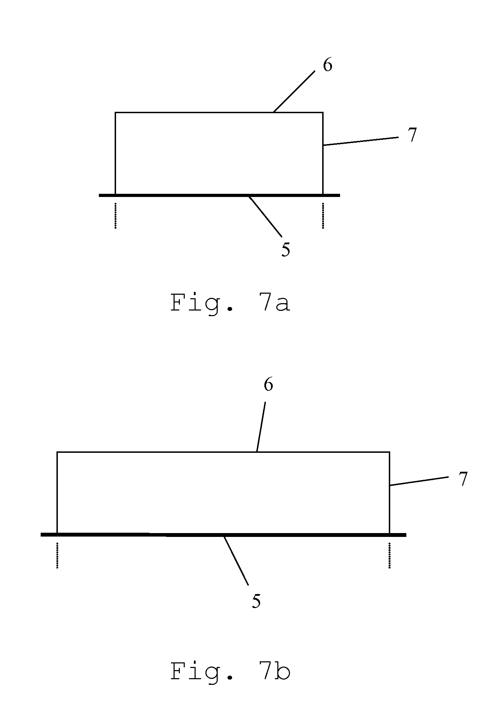

Die **Figuren 7a** und **7b** zeigt das Kühlelement mit den beiden Durchtrittsflächen 5 und 6 und den Seitenflächen 7 in zwei verschiedenen Seitenansichten.

Die Fläche 5 ist die kühlbare optische Durchtrittsfläche.

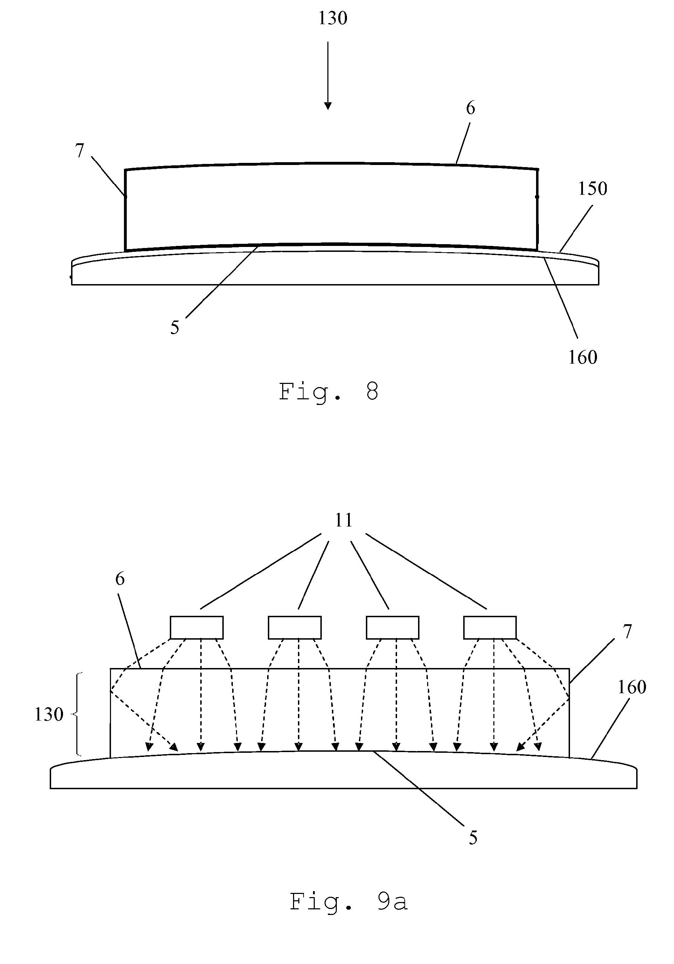

Aus **Figur 8** ist die Flexibilität des Kühlelements 130 erkennbar. Dieses ist im abgebildeten Fall mittels des Tragegurts 150 an der Behandlungsfläche (Haut) 160 fixiert.

Die Durchtrittsflächen 5 und 6 des Kühlelements weisen als funktionale Komponente bauartbedingt eine Flexibilität bei einem durch das Fixieren entstehenden zusätzlichen äußeren Druck auf. Die kühlbare optische Durchtrittsfläche 5 ist auf diese Weise an die Form der Behandlungsfläche 160 anpassbar; die zum Anliegen an der Austrittsseite eines Bestrahlungselements ausgebildete zweite Durchtrittsfläche 6 ist gleichermaßen verformbar.

Die Seitenflächen 7 des Kühlelements 130 dagegen bewahren in der obigen Situation bauartbedingt im wesentlichen ihre Form und weisen als funktionale Komponente eine geringere Verformbarkeit als die Durchtrittsflächen 5 und 6 auf.

Die **Figur 9a** gibt einen möglichen Verlauf der Strahlung innerhalb des Kühlelements (Gel-Pad) 130 wieder. Die von den Lichtquellen 11 emittierte Strahlung tritt durch die Durchtrittsfläche 6 ins Innere des durch Anliegen an die konvex gewölbte Hautoberfläche 160 deformierten Kühlelements 130 ein, wird durch dessen Linseneffekt sowie die Reflektion an der Innenseite der Seitenwände 7 durchmischt und tritt schließlich weitgehend homogenisiert aus der nunmehr gekrümmten kühlbaren optischen Durchtrittsfläche 5 wieder aus.

Je nach konkreter Ausgestaltung und den gegebenen geometrischen Verhältnissen kommt es zu einem Kreuzen der Strahlgänge für die einzelnen Lichtquellen 11 und somit zu einer Durchmischung der Strahlung, was die Homogenisierung unterstützt und ggf. auch bei Ausfall einer Lichtquelle 11 das Entstehen gänzlich unbeleuchteter Zonen verhindert.

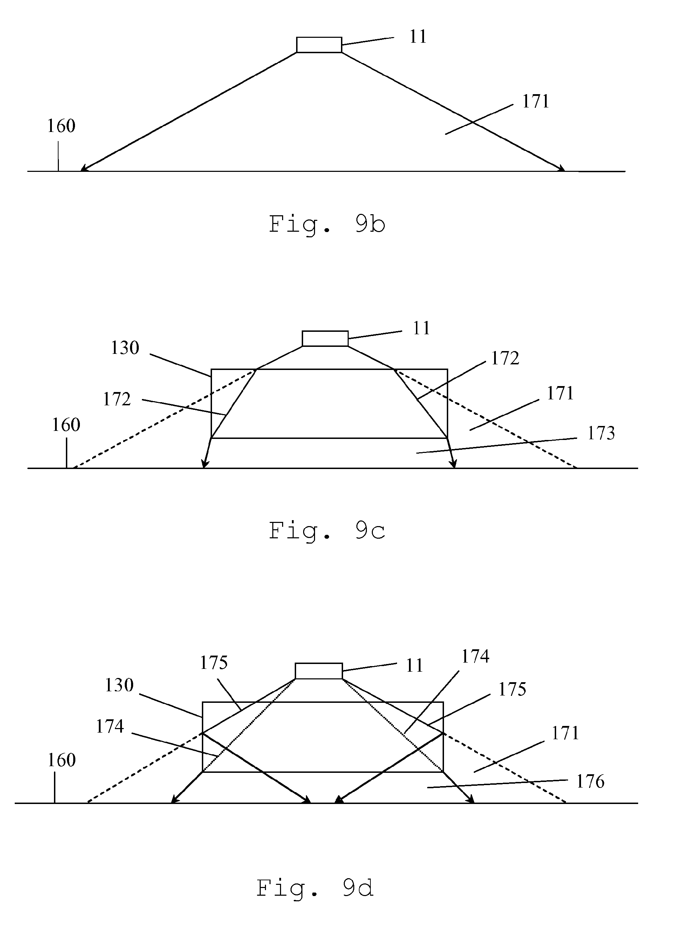

Den **Figuren 9b, 9c** und **9d** ist schematisch den Verlauf der von einer Lichtquelle 11 des Bestrahlungselements emittierten Strahlung zu entnehmen.

Das Ziel der Homogenisierung der Strahlung besteht darin, diese an der kühlbaren optischen Durchtrittsfläche 5 ohne innere Struktur, d.h. mit einem möglichst gleichförmigen Intensitätsverlauf, und einer möglichst senkrecht zur Hautoberfläche 160 bzw. der kühlbaren optischen Durchtrittsfläche 5 erfolgenden Einfallsrichtung bereitzustellen.

In **Figur 9b** trifft das unbeeinflusste Strahlungsfeld 171 auf die Behandlungsfläche 160 auf.

**Figur 9c** illustriert die kollimierende Wirkung (Linseneffekt) des Kühlelements 130. Die das zuvor unbeeinflusste Strahlungsfeld 171 nach außen begrenzenden Lichtstrahlen 172 werden abgelenkt und begrenzen den deutlich schmaleren Lichtkegel des resultierenden Strahlungsfelds 173.

**Figur 9d** stellt die Wirkung der Reflektion der Strahlung an den Innenseiten der Seitenwände des Kühlelements 130 dar.

Die außerhalb des durch die Lichtstrahlen 174 begrenzten Lichtkegels aus der Lichtquelle 11 austretenden Strahlen werden im wesentlichen an den Seitenwänden reflektiert und treffen innerhalb des Lichtkegels des resultierenden Strahlungsfelds 176 auf die Behandlungsfläche 160 auf. Als Beispiel dafür ist der Strahlengang der den Lichtkegel des unbeeinflussten Strahlungsfelds 171 begrenzenden Lichtstrahlen 175 illustriert.

Die in den **Figuren 9c** und **9d** aus Gründen der Anschaulichkeit einzeln beschriebenen Einflüsse auf den Strahlungsgang finden tatsächlich gleichzeitig statt und bewirken gemeinsam eine Veränderung des Strahlquerschnitts. In Abhängigkeit von der exakten Ausbildung des Kühlelements besitzen die obigen Effekte in ihrer Auswirkung jeweils ein unterschiedliches Gewicht.

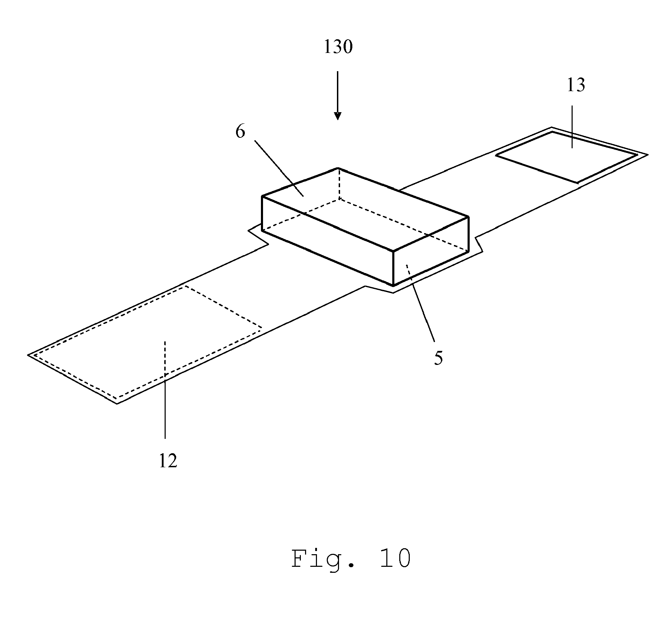

**Figur 10** zeigt ein auf einem Tragegurt aufgeschweißtes Kühlelement 130 mit seinen Durchtrittsflächen 5 und 6 in der Schrägansicht. Der Tragegurt weist zwei Klettverschlüsse 12 und 13 auf.

Die Fläche 5 ist die kühlbare optische Durchtrittsfläche.

Mittels des Tragegurts ist das Kühlelement an der Körperoberfläche des Patienten, beispielsweise am Rumpf oder an den Extremitäten, fixierbar.

In einer nicht dargestellten Ausführung der Erfindung sind mehrere Kühlelemente nebeneinander an einem Tragegurt aufgeschweißt.

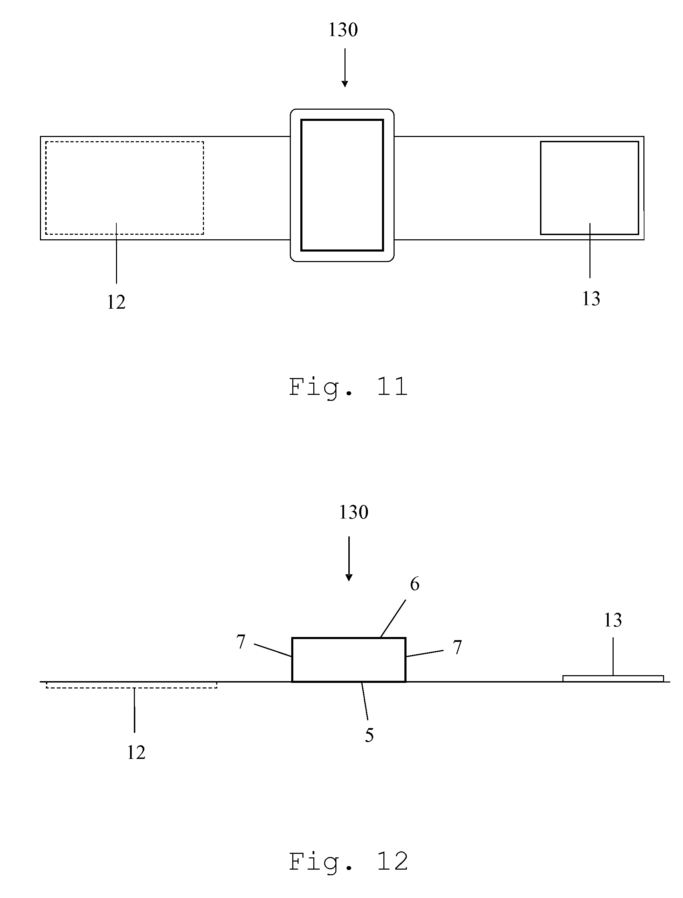

**Figur 11** zeigt ein auf einem mit zwei Klettverschlüssen 12 und 13 versehenen Tragegurt aufgeschweißtes Kühlelement 130 in der Durchtrittsrichtung der emittierten Strahlung.

**Figur 12** zeigt ein auf einem mit zwei Klettverschlüssen 12 und 13 versehenen Tragegurt aufgeschweißtes Kühlelement 130 in der Seitenansicht.

Das Kühlelement weist die Durchtrittsflächen 5 und 6 sowie die Seitenflächen 7 auf. Die Fläche 5 ist die kühlbare optische Durchtrittsfläche.

## Patentansprüche

1. Bestrahlungs-Kühl-Kombination für die Anwendung in einer photodynamischen Therapie - insbesondere einer Applikation eines Photosensitizers -, mit
• einem Bestrahlungselement (120) mit einer Austrittsrichtung für eine zu emittierende Strahlung,
• einem Kühlelement (130), wobei dieses
o zur Anbringung zwischen dem Bestrahlungselement (120) und einer Behandlungsfläche (160) - insbesondere der Hautoberfläche eines Patienten - ausgebildet ist,
o in einer Durchtrittsrichtung durchlässig für die emittierte Strahlung ist,
o eine kühlbare optische Durchtrittsfläche (5) für die Strahlung als eine Anlage an die Behandlungsfläche sowie eine zweite Durchtrittsfläche (6) als Anlage an das Bestrahlungselement (120) aufweist,
• einer ersten Fixiervorrichtung zur definierten Positionierung der Bestrahlungs-Kühl-Kombination gegenüber der Behandlungsfläche (160),
**dadurch gekennzeichnet, dass**
das Kühlelement (130) aufweist
• wenigstens eine Seitenfläche (7), wobei diese
o im wesentlichen senkrecht zu den Durchtrittsflächen (5,6) orientiert ist und
o so ausgebildet ist, dass die Seitenfläche (7) eine geringere Flexibilität als die Durchtrittsflächen (5,6) besitzt,
o mittels einer gegebenen Höhe (9) der Seitenfläche (7) einen Abstand der Durchtrittsflächen (5,6) vordefiniert, wobei dieser unter dem Anliegen eines beim Fixieren entstehenden zusätzlichen äußeren Drucks im wesentlichen konstant bleibt,
○ zur Reflektion der emittierten Strahlung ausgebildet ist,
• ein zum Vorgekühltwerden geeignetes deformierbares -
insbesondere flüssiges oder gelartiges - Füllmedium, wobei das Kühlelement (130) eine infolge der Anbringung an die Behandlungsfläche (160) bewirkbare Deformierbarkeit aufweist, so dass mittels einer divergenzverändernden Wirkung auf einen Strahlquerschnitt der emittierten Strahlung eine Homogenisierung der Strahlung innerhalb des Kühlelements (130) resultiert.

2. Bestrahlungs-Kühl-Kombination nach Anspruch 1,
**gekennzeichnet durch**
eine Steuereinheit (110) zur Begrenzung der Zeitdauer und Intensität einer Bestrahlung sowie zur Auswahl einer oder mehrerer Wellenlängen der **durch** das Bestrahlungselement (120) emittierten Strahlung, sowie insbesondere ein Netzteil (100) zur Spannungsversorgung.

3. Bestrahlungs-Kühl-Kombination nach Anspruch 1 oder 2,
**dadurch gekennzeichnet, dass**
das Bestrahlungselement (120) als LED-Modul mit Vorsatzlinsen ausgebildet ist.

4. Bestrahlungs-Kühl-Kombination nach einem der vorhergehenden Ansprüche,
**dadurch gekennzeichnet, dass**
die wenigstens eine Seitenfläche (7) des Kühlelements (130) zur Erhöhung ihres Reflektionsvermögens poliert, geschliffen, beschichtet oder bedampft ist.

5. Bestrahlungs-Kühl-Kombination nach einem der vorhergehenden Ansprüche,
**dadurch gekennzeichnet, dass**
das Bestrahlungselement (120) zur Emission einer elektromagnetischen Strahlung mit mindestens einer Wellenlänge im Spektrum des sichtbaren Lichts sowie des nahen Infrarot- und Ultraviolettbereichs ausgebildet ist.

6. Bestrahlungs-Kühl-Kombination nach Anspruch 2,
**dadurch gekennzeichnet, dass**
Bestrahlungselement (120), Steuereinheit (110) und Netzteil (100) ausgebildet sind
• jeweils als räumlich trennbare und als Modul ausgebildete Einzelgeräte und/oder
• als eine aus mehreren der genannten Einzelgeräte bestehende Gesamteinheit.

7. Bestrahlungs-Kühl-Kombination nach einem der vorhergehenden Ansprüche,
**dadurch gekennzeichnet, dass**
jeweils ein Kühlelement (130) und ein Bestrahlungselement (120) ein gemeinsames Bestrahlungsmodul bilden, wobei mehrere dieser Bestrahlungsmodule einer gemeinsamen Steuereinheit (110) zuordenbar sind.

8. Bestrahlungs-Kühl-Kombination nach Anspruch 7,
**dadurch gekennzeichnet, dass**
mehrere Bestrahlungsmodule unabhängig voneinander mittels der Steuereinheit (110) regelbar sind.

9. Bestrahlungs-Kühl-Kombination nach einem der vorhergehenden Ansprüche,
**dadurch gekennzeichnet, dass**
das Kühlelement (130) als Gel-Pad mit einer Hülle aus einer ohne zusätzlichen äußeren Druck im wesentlichen formstabilen Kunststoff-Folie ausgebildet ist, insbesondere aus einer Polyurethan-Folie.

10. Bestrahlungs-Kühl-Kombination nach einem der vorhergehenden Ansprüche,
**dadurch gekennzeichnet, dass**
das Kühlelement (130) als Gel-Pad mit einem Füllmedium aus einem viskosen medizinischen Öl mit einer Durchlässigkeit für sichtbares Licht und einem Stockpunkt von unter 0°C ausgebildet ist.

11. Bestrahlungs-Kühl-Kombination nach einem der vorhergehenden Ansprüche,
**dadurch gekennzeichnet, dass**
die Durchtrittsflächen (7) des Kühlelements (130) bauartbedingt nach einer Anbringung der Bestrahlungs-Kühl-Kombination an der Behandlungsfläche (160) deformierbar sind, insbesondere aufgrund
• des Verhältnisses der Seitenlängen (9,10a,10b) des Kühlelements (130) zueinander, wobei insbesondere die Höhe (9) der Seitenflächen (7) kleiner ist als die Länge der kürzesten geometrischen Achse der Durchtrittsflächen (5,6),
• des Verhältnisses der Flächeninhalte der Durchtrittsflächen (5,6) und der Seitenflächen (7) des Kühlelements (130) zueinander, wobei insbesondere die Summe der Flächeninhalte der Seitenflächen (7) geringer ist als die Summe der Flächeninhalte der Durchtrittsflächen (5,6),
• von unterschiedlichen Wandstärken der Seiten- und Durchtrittsflächen, wobei insbesondere die Wandstärke der Seitenflächen (7) größer als die Wandstärke der Durchtrittsflächen (5,6) ist, und/oder
• von unterschiedlichen Materialien der Seiten- und Durchtrittsflächen, wobei insbesondere das Material der Seitenfläche (7) an sich eine geringe Flexibilität als das Material der Durchtrittsflächen (5,6) aufweist.

12. Bestrahlungs-Kühl-Kombination nach einem der vorhergehenden Ansprüche,
**gekennzeichnet durch**
eine zweite Fixierungsvorrichtung (140) zur Anbringung des Bestrahlungselements (120) an das Kühlelement (130), insbesondere wobei diese als Spange oder Rahmen ausgebildet ist.

13. Bestrahlungs-Kühl-Kombination nach einem der vorhergehenden Ansprüche,
**gekennzeichnet durch**
einen Gurt (150) zur Anbringung wenigstens des Kühlelements (130) an der Behandlungsfläche (160), insbesondere einen Polymer-Gurt, insbesondere wobei das Kühlelement (130) mit dem Gurt (150) verschweißt ist.

14. Verfahren zum Erzeugen eines homogenisierten Strahlungsfeldes an einer kühlbaren optischen Durchtrittsfläche (5), insbesondere mit einer Bestrahlungs-Kühl-Kombination nach einem der vorangehenden Ansprüche, mit einem
o Erzeugen eines Strahlungsfelds durch eine Vielzahl von diskreten Strahlungsquellen (1),
o Leiten der Strahlung durch ein Kühlelement wobei die optische Durchtrittsfläche eine Außenfläche des Kühlelements (130) darstellt,
**dadurch gekennzeichnet, dass**
das Strahlungsfeld (170) durch ein
• Veränderung der Divergenz der Strahlung mittels eines durch eine Deformation des Kühlelements (130) bewirkten Linseneffekts,
• Reflektieren der Strahlung an den Seitenflächen (7) des Kühlelements (130),
homogenisiert wird, insbesondere mit einem zumindest teilweisen Durchmischen des Strahlgangs.

15. Verfahren nach Anspruch 14,
**gekennzeichnet durch**
ein Vorgekühltwerden des kühl-optischen Elements, insbesondere auf eine Temperatur von 4-6°C, unmittelbar vor dem Anbringen des Kühlelements (130) an eine Behandlungsfläche (160).
